# FASCICULE DE BREVET EUROPEEN

(11) **EP 4 623 212 B1**
(45) Date de publication et mention de la délivrance du brevet: **11.11.2026**
(21) Numéro de dépôt: 22830901.9
(22) Date de dépôt: 22.11.2022
(51) Int. Cl.: F16C 11/04, G01N 33/28

(54) **SYSTÈME D'ACTIONNEMENT POUR DISPOSITIF DE CARACTÉRISATION THERMODYNAMIQUE**
BETÄTIGUNGSSYSTEM FÜR EINE THERMODYNAMISCHE CHARAKTERISIERUNGSVORRICHTUNG
ACTUATION SYSTEM FOR A THERMODYNAMIC CHARACTERIZATION DEVICE

(43) Date de publication de la demande: 01.10.2025
(73) Titulaire: Irian Innovation, 82200 Moissac (FR)
(72) Inventeur: BICKERT, Jacques, 64110 UZOS (FR); DELHOSTE, Thomas, ASSAT 64510 (FR)
(74) Mandataire: IPAZ
(86) Numéro de dépôt international: PCT/FR2022/052148
(87) Numéro de publication internationale: WO 2024/110700

(56) Documents cités:
- FR-A1- 3 001 546
- US-A- 3 838 571
- ANONYMOUS: "Linkages", 18 September 2020 (2020-09-18), pages 1 - 4, XP093046196, Retrieved from the Internet <URL:https://web.archive.org/web/20200918144729if_/https://www.notesandsketches.co.uk/PDF/Linkages.pdf> [retrieved on 20230511]

## Description

### Domaine technique

L'invention se rapporte au domaine des systèmes de transmission de mouvement du type à bras de levier.

L'invention présente un intérêt particulier dans le secteur de l'analyse thermodynamique d'un fluide, par exemple une huile prélevée lors d'un forage d'exploration ou d'exploitation.

### État de la technique antérieure

Les équipements conventionnels pour l'analyse thermodynamique de fluide présentent un volume et une masse ne permettant pas leur transport sur site de prélèvement, rendant nécessaire l'envoi d'échantillons vers des laboratoires d'analyse distants.

Des tentatives ont été menées pour miniaturiser ces équipements afin de pouvoir réaliser des analyses *in situ.*

Dans ce contexte, il a été proposé un dispositif compact, décrit dans le document FR3001546A1, doté d'une chambre de compression prévue pour recevoir un volume de fluide à analyser n'excédant pas 1 cm³. Ce dispositif comprend un piston permettant de modifier le volume de la chambre et un système d'actionnement du piston incluant un bras de levier afin de démultiplier l'effort transmis au piston.

Compte tenu de la pression du fluide dans la chambre qui peut atteindre 1000 bars et des efforts qu'il est nécessaire d'exercer sur le piston, le pivotement d'un tel bras de levier induit des contraintes transversales sur le piston qui entraînent une usure prématurée du dispositif et des imprécisions de mesure.

Des systèmes de transmission de mouvement du type à bras de levier sont généralement connus, voir par exemple https://web.archive.org/web/20200918144729if_/https:// www.notesandsketches.co.uk/PDF/Linkages.pdf.

### Exposé de l'invention

L'invention a pour objectif de procurer un système d'actionnement permettant de réduire l'usure du dispositif et/ou d'améliorer le cas échéant la précision des mesures.

Un but particulier de l'invention est de procurer une solution compatible avec un dispositif de caractérisation thermodynamique compact.

A cet effet, l'invention a pour objet un dispositif comprenant une structure fixe, une structure mobile le long d'une direction longitudinale et un système d'actionnement de la structure mobile. Selon l'invention, le système d'actionnement comprend un bras de levier et une bielle, le bras de levier comprenant :
- une première partie reliée à une première structure formée par l'une parmi la structure mobile et la structure fixe selon une liaison définissant un degré de liberté en rotation autour d'un premier axe de rotation perpendiculaire à la direction longitudinale,
- une deuxième partie reliée à la bielle selon une liaison définissant un degré de liberté en rotation autour d'un deuxième axe de rotation parallèle au premier axe de rotation, la bielle étant reliée à une deuxième structure formée par l'autre parmi la structure mobile et la structure fixe selon une liaison définissant un degré de liberté en rotation autour d'un troisième axe de rotation parallèle au deuxième axe de rotation.

Un tel système d'actionnement permet d'améliorer la répartition des efforts exercés sur la structure mobile lors du déplacement du bras de levier et, notamment, de réduire ou d'annuler les contraintes transversales exercées sur la structure mobile.

L'invention permet ainsi d'éviter une usure prématurée du dispositif.

L'invention permet aussi d'améliorer la précision du déplacement de la structure mobile, ce qui permet le cas échéant d'augmenter la précision de mesures associées à un tel déplacement.

Selon l'invention, la bielle comprend une première extrémité définissant le deuxième axe de rotation et une deuxième extrémité définissant le troisième axe de rotation, la première extrémité de la bielle étant en appui sur une surface de pivotement formée par la deuxième partie du bras de levier, la deuxième extrémité de la bielle étant en appui sur une surface de pivotement formée par ladite deuxième structure.

De préférence, la surface de pivotement formée par la deuxième partie du bras de levier et la surface de pivotement de la deuxième structure sont disposées en regard l'une par rapport à l'autre.

Dans un mode de réalisation, le dispositif comprend un axe tel qu'une goupille définissant le premier axe de rotation.

Ledit axe est de préférence solidaire de ladite première structure, plus préférentiellement au moins en translation le long de la direction longitudinale lorsque la première structure est formée par la structure mobile.

La première partie du bras de levier peut comprendre une surface de pivotement configurée pour venir en appui sur ledit axe.

Dans un mode de réalisation, la surface de pivotement formée par la première partie du bras de levier est configurée pour exercer sur ledit axe un effort ayant une composante orientée dans un premier sens selon la direction longitudinale, la surface de pivotement formée par la deuxième partie du bras de levier étant configurée pour exercer sur la bielle un effort ayant une composante orientée dans un deuxième sens selon la direction longitudinale.

Dans un mode de réalisation, le système d'actionnement comprend une pièce d'entraînement, le bras de levier comprenant une troisième partie reliée à cette pièce d'entraînement selon une liaison définissant un degré de liberté en rotation autour d'un quatrième axe de rotation parallèle au premier axe de rotation.

De préférence, le premier axe de rotation, le deuxième axe de rotation et le quatrième axe de rotation appartiennent à un même plan.

Dans un mode de réalisation, la pièce d'entraînement est mobile en translation le long d'un axe de déplacement parallèle à la direction longitudinale.

Dans un mode de réalisation, le système d'actionnement comprend un moteur configuré pour déplacer le bras de levier en rotation simultanément autour du premier axe de rotation et du deuxième axe de rotation.

Le système d'actionnement de l'invention permet de miniaturiser le moteur et par conséquent de gagner en encombrement et masse.

Dans un mode de réalisation, le moteur est configuré pour déplacer le bras de levier en rotation autour du premier axe de rotation et du deuxième axe de rotation par l'intermédiaire de la pièce d'entraînement.

Dans un mode de réalisation préféré, la structure mobile forme ladite première structure et dans lequel la structure fixe forme ladite deuxième structure.

Le dispositif est de préférence destiné à la caractérisation thermodynamique d'un fluide.

Dans un mode de réalisation, le dispositif comprend une chambre apte à recevoir le fluide, la structure mobile étant configurée pour pouvoir modifier le volume de la chambre.

D'autres avantages et caractéristiques de l'invention apparaîtront à la lecture de la description détaillée, non limitative, qui suit.

### Brève description des dessins

La description détaillée qui suit fait référence aux dessins annexés sur lesquels :
Fig. 1 est une vue partielle en coupe longitudinale d'un dispositif de caractérisation thermodynamique conforme à l'invention, selon un plan de coupe passant par un axe de translation d'un piston du dispositif, le dispositif comprenant un système d'actionnement du piston incluant un bras de levier ;
Fig. 2 est un agrandissement d'une partie du dispositif de la figure 1, centré sur le piston, les pièces coupées étant représentées sans hachures afin de faciliter la visualisation des références ;
Fig. 3 est une vue schématique du bras de levier du dispositif de la figure 1, le bras de levier étant dans une première position par rapport à une structure fixe du dispositif ;
Fig. 4 est une vue schématique du bras de levier du dispositif de la figure 1, le bras de levier étant dans une deuxième position par rapport à ladite structure fixe.

### Description détaillée de modes de réalisation

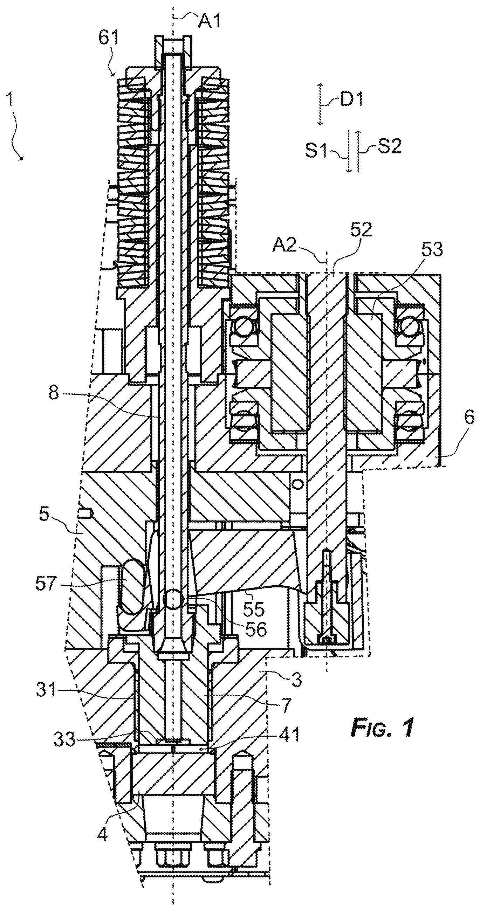

Il est représenté sur la figure 1 un dispositif 1 conforme à l'invention, destiné à la caractérisation thermodynamique d'un fluide.

Le dispositif 1 comprend une structure fixe et une structure mobile, relativement à la structure fixe, selon une direction longitudinale D1. La direction D1 définit un premier sens S1 de déplacement de la structure mobile, allant du haut vers le bas de la figure 1, et un deuxième sens S2 allant du bas vers le haut de la figure 1.

Dans cet exemple non limitatif, la structure fixe comprend différentes pièces 3-6 assemblées les unes aux autres de la manière illustrée sur la figure 1, la structure mobile comprenant un piston 7 et un tirant 8 solidaires l'un de l'autre en translation longitudinale, c'est-à-dire selon la direction D1.

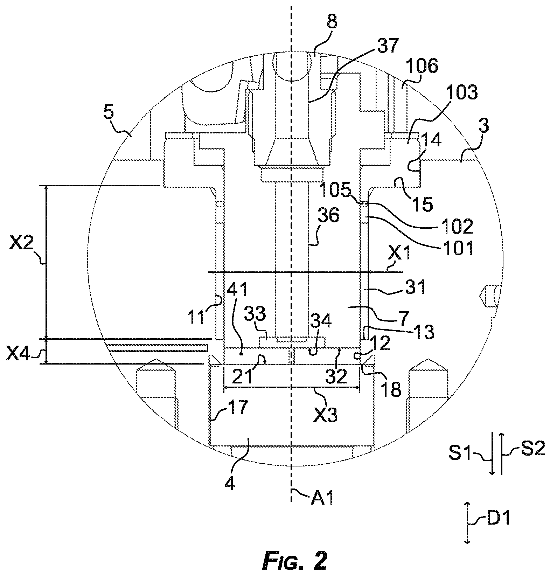

En référence à la figure 2, qui montre un agrandissement d'une partie de ces structures fixe et mobile, centré sur le piston 7, la pièce 3 de la structure fixe, aussi appelée « corps », comprend une ouverture qui la traverse de part en part selon la direction D1, de manière à s'étendre autour d'un axe A1.

L'ouverture du corps 3 comprend un alésage 11 d'axe A1 et de diamètre X1 qui s'étend sur une portion longitudinale de dimension X2, ainsi qu'un alésage 12 d'axe A1 et de diamètre X3 qui s'étend sur une portion longitudinale de dimension X4.

Dans cet exemple nullement limitatif, les dimensions X1, X2, X3 et X4 sont respectivement égales à 28 mm, 28 mm, 25 mm et 4,55 mm.

Le diamètre X3 de l'alésage 12 étant inférieur au diamètre X1 de l'alésage 11, le corps 3 forme un épaulement 13 définissant une surface d'appui annulaire qui s'étend dans un plan perpendiculaire à la direction D1.

Dans cet exemple, l'ouverture du corps 3 comprend un lamage 14 par lequel l'alésage 11 débouche au niveau d'une première extrémité longitudinale du corps 3, aussi appelée « extrémité supérieure ». Le lamage 14 forme un épaulement 15 qui définit une surface d'appui annulaire s'étendant dans un plan perpendiculaire à la direction D1.

L'ouverture du corps 3 comprend aussi un lamage 17 par lequel l'alésage 12 débouche au niveau d'une deuxième extrémité longitudinale du corps 3, aussi appelée « extrémité inférieure ». Le lamage 17 forme un épaulement 18 qui définit une surface d'appui annulaire s'étendant dans un plan perpendiculaire à la direction D1.

La pièce 4 de la structure fixe, aussi appelée « hublot » car elle est configurée pour permettre une visualisation de l'intérieur de la chambre 41, est logée dans le lamage 17 du corps 3, de sorte qu'une surface 21 du hublot 4 soit en appui sur la surface annulaire formée par l'épaulement 18.

Le hublot 4 obture ainsi l'ouverture du corps 3 au niveau de son extrémité inférieure.

Le piston 7 est reçu dans un logement de la structure fixe ici formé par les alésages 11 et 12 et par le lamage 14 du corps 3.

Un joint 31 d'étanchéité annulaire est disposé dans l'alésage 11 de manière à s'étendre radialement entre le piston 7 et la surface du corps 3 qui forme cet alésage 11.

Le piston 7 comprend une surface 32 disposée en regard de la surface 21 du hublot 4.

Dans cet exemple, un capteur 33 est logé dans le piston 7 de manière à présenter une surface 34 qui affleure la surface 32 du piston 7. De manière non limitative, le capteur 33 est équipé d'instruments incluant une jauge de déformation (non représentée) et d'une sonde à résistance de platine (non représentée) destinées à mesurer la pression et la température du fluide dans la chambre 41.

Ces instruments sont raccordés à un module (non représenté) de conditionnement de signal par des câbles (non représentés) qui traversent des orifices 36 et 37 réalisés respectivement dans le piston 7 et le tirant 8.

Le dispositif 1 forme ainsi une chambre 41 annulaire qui est délimitée radialement par la surface du corps 3 formant l'alésage 12. Longitudinalement, la chambre 41 est délimitée d'une part, par la surface 21 du hublot 4 et, d'autre part, par la surface 32 du piston 7 et la surface 34 du capteur 33, les surfaces 21, 32 et 34 étant dans cet exemple perpendiculaires à la direction D1.

Le piston 7 est monté coulissant selon la direction D1, et par suite selon l'axe A1 le long duquel il s'étend.

Les figures 1 et 2 montrent le piston 7 et le tirant 8 dans une première position, dans laquelle la chambre 41 présente un volume ayant une première valeur. Un déplacement du piston 7 et du tirant 8 en direction du hublot 4, jusqu'à une deuxième position (non représentée), permet de réduire le volume de la chambre 41 jusqu'à une deuxième valeur inférieure à la première valeur.

Dans cet exemple nullement limitatif, le volume de la chambre est de l'ordre de 1,5 cm³ lorsque le piston 7 est dans la première position et est sensiblement nul lorsque le piston 7 est dans la deuxième position, la course du piston 7 entre la première et la deuxième position étant d'environ 3 mm.

La chambre 41 forme ainsi une chambre de compression apte à contenir un fluide sous pression.

Dans l'exemple de la figure 1, la commande du piston 7 est réalisée à l'aide d'un système d'actionnement comprenant un moteur électrique équipé d'un codeur (non représenté).

Le système d'actionnement comprend un mécanisme de transmission configuré pour transformer un mouvement rotatif d'un arbre moteur (non représenté) en une translation du piston 7 selon D1.

Dans cet exemple non limitatif, le mécanisme de transmission comprend une vis 52 configurée pour être entraînée en translation suivant un axe A2, parallèle à l'axe A1, sous l'action d'un écrou 53. L'arbre moteur entraîne une vis (non représentée), qui coopère avec une roue (non représentée) solidaire de l'écrou 53, de manière à former un engrenage du type roue et vis sans fin.

La vis 52 coopère avec l'écrou 53 qui est solidaire de la pièce 6 de la structure fixe, de sorte qu'une rotation de l'écrou 53 autour de l'axe A2 entraîne une translation de cette vis 52 selon D1.

Le mécanisme de transmission comprend dans cet exemple un sous-système incluant un bras de levier 55, un axe 56 et une bielle 57, configuré pour transmettre au piston 7 le mouvement de translation de la vis 52 par l'intermédiaire d'une rotation du bras de levier 55. Ce sous-système de transmission, décrit plus en détails plus loin ci-dessous, permet de démultiplier l'effort transmis au piston 7 et de réduire notamment la taille du moteur.

Le dispositif 1 comprend par ailleurs un ressort 61 de rattrapage de jeu formé par un empilement de rondelles coniques qui sont configurées pour exercer un effort de traction sur le tirant 8, et par suite sur le piston 7, dans le sens S2 de la direction D1.

Ainsi, lorsque le piston 7 est déplacé dans le sens S1 sous l'action du bras de levier 55, la structure mobile comprime le ressort 61 qui est dimensionné pour maintenir une charge sur cette structure mobile et sur le bras de levier 55, afin d'éviter qu'un jeu présent dans le mécanisme de transmission n'entraîne des erreurs de mesure.

Le dispositif 1 comprend par ailleurs des circuits et des vannes, non représentés, prévus d'une part pour introduire un échantillon de fluide dans la chambre 41 en vue par exemple d'une analyse et, d'autre part, pour évacuer le fluide de la chambre 41 notamment en fin d'analyse.

De manière connue en soi, le dispositif 1 comprend d'autres organes, non représentés, incluant de façon non limitative :
- un système de chauffage du fluide contenu dans la chambre 41, et/ou
- une caméra haute définition pour étudier des changements de phase et/ou des sédimentations du fluide contenu dans la chambre 41, et/ou
- un système d'agitation du fluide contenu dans la chambre 41, par exemple par vibrations, et/ou
- un système de refroidissement du fluide contenu dans la chambre 41, et/ou
- un gazomètre relié à la chambre 41 afin de réaliser des essais complémentaires.

De manière générale, le dispositif 1 permet de réaliser des analyses thermodynamiques d'un fluide tel qu'une huile hydrocarbonée, en particulier par analyse du comportement de phase lors d'une réduction du volume de la chambre 41 sous l'action d'un déplacement du piston 7.

De telles analyses peuvent être réalisées directement sur site de forage pétrolier, par exemple, compte tenu de l'encombrement et de la masse du dispositif 1 qui facilitent son transport. Dans cet exemple, le dispositif 1 présente un encombrement inférieur à 0,1 m³ et une masse globale d'une quinzaine de kg.

L'invention se rapporte plus spécifiquement au sous-système de transmission de la translation de la vis 52 au piston 7 par le bras de levier 55.

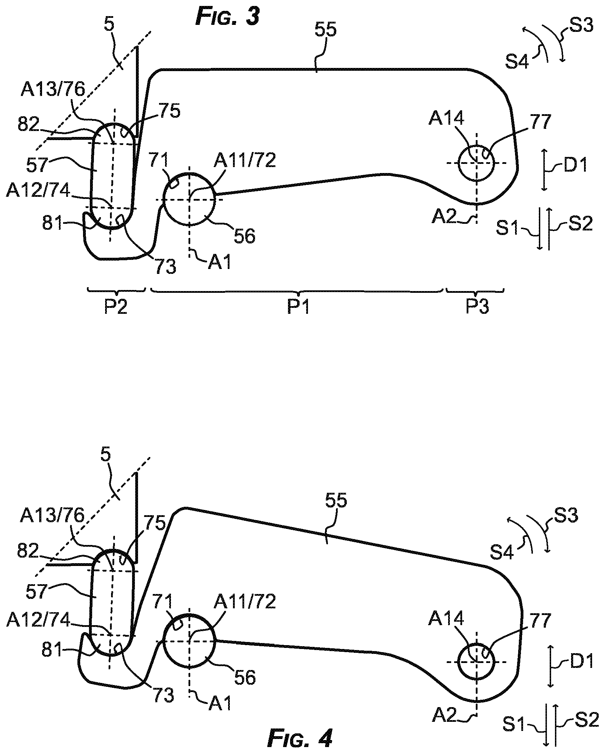

Les figures 3 et 4 montrent le bras de levier 55, l'axe 56, la bielle 57 ainsi qu'une partie de la pièce 5 de la structure fixe, aussi appelée « support ».

Sur la figure 3, le bras de levier 55, l'axe 56 et la bielle 57 sont dans une première configuration par rapport au support 5, identique à la configuration de la figure 1. Dans cette première configuration, le piston 7 et le tirant 8 sont dans ladite première position et la vis 52 est également dans une première position.

Sur la figure 4, le bras de levier 55, l'axe 56 et la bielle 57 sont dans une deuxième configuration par rapport au support 5. Dans cette deuxième configuration, le piston 7 et le tirant 8 sont dans ladite deuxième position et la vis 52 est également dans une deuxième position.

En référence à la figure 3, sur laquelle le bras de levier 55 est représenté en vue de face qui correspond sensiblement à une projection du bras de levier 55 dans un plan parallèle à D1, le bras de levier 55 se présente sous forme d'une pièce allongée définissant une partie centrale P1, aussi appelée « première partie », une partie distale P2, aussi appelée « deuxième partie », et une partie proximale P3, aussi appelée « troisième partie ».

La partie centrale P1 du bras de levier 55 comprend une surface 71 curviligne. Dans cet exemple non limitatif, la surface 71 s'étend circonférentiellement autour d'un axe de rotation A11 perpendiculaire à la direction D1, de manière à définir un arc de cercle ayant un centre 72 par lequel passe l'axe de rotation A11 et présentant un angle d'environ 180°.

La partie distale P2 du bras de levier 55 forme un doigt définissant une surface 73 curviligne. Dans cet exemple non limitatif, la surface 73 s'étend circonférentiellement autour d'un axe de rotation A12 perpendiculaire à la direction D1, de manière à définir un arc de cercle ayant un centre 74 par lequel passe l'axe de rotation A12 et présentant un angle d'environ 150°.

Le support 5 comprend lui aussi une surface 75 curviligne. Dans cet exemple non limitatif, la surface 75 s'étend circonférentiellement autour d'un axe de rotation A13 perpendiculaire à la direction D1, de manière à définir un arc de cercle ayant un centre 76 par lequel passe l'axe de rotation A13 et présentant un angle d'environ 150°.

Dans la configuration de la figure 3, la surface 73 formée par le bras de levier 55 et la surface 75 formée par le support 5 sont disposées en regard l'une de l'autre.

Dans la configuration de la figure 3, les surfaces 71 et 73 sont configurées de sorte qu'il existe un plan perpendiculaire à la direction D1 pouvant être positionné de sorte que la surface 71 s'étende d'un premier côté de ce plan et la surface 73 s'étende d'un deuxième côté de ce plan. Autrement dit, relativement à la direction D1, la surface 71 s'étend d'un premier côté par rapport à son centre 72 et la surface 73 s'étend d'un deuxième côté par rapport à son centre 74.

Ainsi, relativement à la direction D1, les surfaces 71 et 75 s'étendent chacune d'un même côté par rapport à leur centre 72 et 76 respectif, la surface 73 s'étendant du côté opposé par rapport à son centre 74.

La partie proximale P3 du bras de levier 55 comprend par ailleurs une surface 77 circulaire s'étendant autour d'un axe de rotation A14 perpendiculaire à la direction D1.

Dans cet exemple, l'axe 56 est une pièce globalement cylindrique, du type goupille, solidaire du tirant 8 et donc du piston 7. L'axe 56 est configuré pour ne pas obturer totalement l'orifice 37 du tirant 8, afin de permettre le passage des câbles de raccordement du capteur 33. Dans cet exemple particulier, l'axe 56 est pourvu d'un perçage permettant le passage de tels câbles.

L'axe 56 s'étend le long d'un axe fictif correspondant à l'axe de rotation A11 et forme une surface d'appui s'étendant circonférentiellement autour de l'axe de rotation A11.

La surface d'appui de l'axe 56 est configurée pour coopérer avec la surface 71 du bras de levier 55, aussi appelée « surface de pivotement », de manière à autoriser un pivotement du bras de levier 55 autour de l'axe de rotation A11.

Le bras de levier 55 coopère ainsi par sa surface de pivotement 71 avec la surface d'appui de l'axe 56, de manière à former avec celui-ci une liaison pivot définissant un degré de liberté en rotation autour de l'axe de rotation A11.

Concernant la bielle 57, celle-ci comprend deux extrémités 81 et 82 formant chacune une surface d'appui qui s'étend circonférentiellement autour de l'axe de rotation A12 ou A13, respectivement.

Le bras de levier 55 coopère par sa surface de pivotement 73 avec la surface d'appui formée par l'extrémité 81 de la bielle 57, de manière à former avec la bielle 57 une liaison pivot définissant un degré de liberté en rotation autour de l'axe de rotation A12.

La bielle 57 coopère par la surface d'appui formée par son extrémité 82 avec la surface 75 du support 5, aussi appelée « surface de pivotement », de manière à former avec le support 5 une liaison pivot définissant un degré de liberté en rotation autour de l'axe de rotation A13.

La bielle 57 est ainsi montée flottante entre le support 5 et le bras de levier 55.

Le bras de levier 55 coopère par ailleurs par sa surface 77 avec un axe (non représenté) solidaire de la vis 52, de manière à former avec celle-ci une liaison pivot définissant un degré de liberté en rotation autour de l'axe de rotation A14.

Dans cet exemple non limitatif, les axes de rotation A11, A12 et A14 sont parallèles entre eux et appartiennent à un même plan sécant à la direction D1, cela dans la configuration de la figure 3, dans celle de la figure 4 et dans toute configuration intermédiaire entre celles des figures 3 et 4.

L'axe de rotation A11 est par ailleurs sécant à l'axe A1 de translation du piston 4, dans les configurations des figures 3 et 4 ainsi que dans les configurations intermédiaires.

De même, l'axe de rotation A14 est sécant à l'axe A2 de translation de la vis 52, dans les configurations des figures 3 et 4 ainsi que dans les configurations intermédiaires.

Pour passer de la configuration de la figure 3 à celle de la figure 4, la vis 52 est déplacée en translation selon l'axe A2, dans le sens S1, sous l'action du moteur.

Lors d'une telle translation de la vis 52, l'extrémité distale P2 du bras de levier 55 prend appui sur le support 5 par l'intermédiaire de la bielle 57, entraînant d'une part une rotation du bras de levier 55 dans un sens S3 simultanément autour des axes de rotation A11, A12 et A14 et, d'autre part, un déplacement de l'axe 56 dans le sens S1 qui déplace le tirant 8 et le piston 7 de la première position vers la deuxième position.

Lors d'un tel pivotement du bras de levier 55, celui-ci exerce d'une part sur l'axe 56 un effort ayant une composante orientée dans le sens S1, via la surface de pivotement 71, et, d'autre part, sur la bielle 57 et par suite sur le support 5 un effort ayant une composante orientée dans le sens S2, via la surface de pivotement 73.

Le pivotement de la bielle 57 permet d'éviter que le bras de levier 55 ne tende à éloigner l'axe de rotation A11 de l'axe de translation A1 lors du pivotement du bras de levier 55. Le pivotement de la bielle 57 tend en effet à préserver la distance entre les axes de rotation A11, A12 et A14 projetée dans un plan perpendiculaire à la direction D1 et permet ainsi d'éviter que les axes de rotation A11 et A14 ne se déplacent suivant une trajectoire elliptique lors de la translation du piston 7.

Inversement, pour passer de la configuration de la figure 4 à celle de la figure 3, la vis 52 est déplacée en translation selon l'axe A2, dans le sens S2, sous l'action du moteur, entraînant un pivotement du bras de levier dans un sens S4 autour des axes de rotation A11, A12 et A14. Le piston 7 et le tirant 8 sont entraînés en translation le long de l'axe A1 dans le sens S2 sous l'action du ressort 61 et le cas échéant sous l'effet de la pression dans la chambre 41.

Dans l'exemple particulier de la figure 1, le système d'actionnement est configuré de sorte qu'une translation de la vis 52 de 14 mm dans le sens S1 entraîne une translation du piston 7 dans le sens S1 d'environ 3 mm, soit une démultiplication d'environ 4,7, ainsi qu'un pivotement de la bielle 57 d'environ 0,5°.

Bien entendu, de nombreuses variantes peuvent être apportées au dispositif décrit ci-dessus. Par exemple, dans un mode de réalisation non représenté, les organes de liaison du bras de levier à la structure fixe d'une part et à la structure mobile d'autre part peuvent être inversés, de sorte que le bras de levier peut être relié à la structure fixe, par exemple au support 5, selon une liaison pivot classique et être reliée à la structure mobile, par rapport au tirant 8, à l'aide d'un organe tel que la bielle 57 formant un double pivot.

Pour autre exemple, le dispositif peut comprendre des organes définissant des degrés de liberté additionnels par rapport à ceux décrits ci-dessus. Ainsi, dans un mode de réalisation non représenté, le bras de levier 55 peut être relié à la vis 52 par l'intermédiaire d'un organe formant un double pivot, tel que la bielle 57.

## Revendications

1. Dispositif (1) comprenant une structure fixe (3-6), une structure (7, 8) mobile le long d'une direction longitudinale (D1) et un système d'actionnement de la structure mobile (7, 8), **caractérisé en ce que** le système d'actionnement comprend un bras de levier (55) et une bielle (57), le bras de levier (55) comprenant :
- une première partie (P1) reliée à une première structure formée par l'une parmi la structure mobile (7, 8) et la structure fixe (3-6) selon une liaison définissant un degré de liberté en rotation autour d'un premier axe de rotation (A11) perpendiculaire à la direction longitudinale (D1),
- une deuxième partie (P2) reliée à la bielle (57) selon une liaison définissant un degré de liberté en rotation autour d'un deuxième axe de rotation (A12) parallèle au premier axe de rotation (A11), la bielle (57) étant reliée à une deuxième structure formée par l'autre parmi la structure mobile (7, 8) et la structure fixe (3-6) selon une liaison définissant un degré de liberté en rotation autour d'un troisième axe de rotation (A13) parallèle au deuxième axe de rotation (A12),
la bielle (57) comprenant une première extrémité (81) définissant le deuxième axe de rotation (A12) et une deuxième extrémité (82) définissant le troisième axe de rotation (A13), la première extrémité (81) de la bielle (57) étant en appui sur une surface de pivotement (73) formée par la deuxième partie (P2) du bras de levier (55), la deuxième extrémité (82) de la bielle (57) étant en appui sur une surface de pivotement (75) formée par ladite deuxième structure (5).

2. Dispositif (1) selon la revendication 1, dans lequel la surface de pivotement (73) formée par la deuxième partie (P2) du bras de levier (55) et la surface de pivotement (75) de la deuxième structure (5) sont disposées en regard l'une par rapport à l'autre.

3. Dispositif (1) selon la revendication 1 ou 2, comprenant un axe (56) tel qu'une goupille définissant le premier axe de rotation (A11), cet axe (56) étant solidaire de ladite première structure (7, 8).

4. Dispositif (1) selon la revendication 3, dans lequel la première partie (P1) du bras de levier (55) comprend une surface de pivotement (71) configurée pour venir en appui sur ledit axe (56).

5. Dispositif (1) selon la revendication 4, dans lequel la surface de pivotement (71) formée par la première partie (P1) du bras de levier (55) est configurée pour exercer sur ledit axe (56) un effort ayant une composante orientée dans un premier sens (S1) selon la direction longitudinale (D1), la surface de pivotement (73) formée par la deuxième partie (P2) du bras de levier (55) étant configurée pour exercer sur la bielle (57) un effort ayant une composante orientée dans un deuxième sens (S2) selon la direction longitudinale (D1).

6. Dispositif (1) selon l'une quelconque des revendications 1 à 5, dans lequel le système d'actionnement comprend une pièce d'entraînement (52), le bras de levier (55) comprenant une troisième partie (P3) reliée à cette pièce d'entraînement (52) selon une liaison définissant un degré de liberté en rotation autour d'un quatrième axe de rotation (A14) parallèle au premier axe de rotation (A11).

7. Dispositif (1) selon la revendication 6, dans lequel le premier axe de rotation (A11), le deuxième axe de rotation (A12) et le quatrième axe de rotation (A14) appartiennent à un même plan.

8. Dispositif (1) selon la revendication 6 ou 7, dans lequel la pièce d'entraînement (52) est mobile en translation le long d'un axe de déplacement (A2) parallèle à la direction longitudinale (D1).

9. Dispositif (1) selon l'une quelconque des revendications 1 à 8, dans lequel le système d'actionnement comprend un moteur configuré pour déplacer le bras de levier (55) en rotation simultanément autour du premier axe de rotation (A11) et du deuxième axe de rotation (A12).

10. Dispositif (1) selon la revendication 9 incluant les caractéristiques de la revendication 6, dans lequel le moteur est configuré pour déplacer le bras de levier (55) en rotation autour du premier axe de rotation (A11) et du deuxième axe de rotation (A12) par l'intermédiaire de la pièce d'entraînement (52).

11. Dispositif (1) selon l'une quelconque des revendications 1 à 10, dans lequel la structure mobile (7, 8) forme ladite première structure et dans lequel la structure fixe (3-6) forme ladite deuxième structure.

12. Dispositif (1) selon l'une quelconque des revendications 1 à 11, destiné à la caractérisation thermodynamique d'un fluide, le dispositif (1) comprenant une chambre (41) apte à recevoir le fluide, la structure mobile (7, 8) étant configurée pour pouvoir modifier le volume de la chambre (41).

## Patentansprüche

1. Vorrichtung (1), umfassend eine feste Struktur (3-6), eine entlang einer Längsrichtung (D1) bewegliche Struktur (7, 8) und ein Betätigungssystem der beweglichen Struktur (7, 8), **dadurch gekennzeichnet, dass** das Betätigungssystem einen Hebelarm (55) und eine Pleuelstange (57) umfasst, der Hebelarm (55) umfassend:
- einen ersten Teil (P1), der mit einer ersten Struktur verbunden ist, die durch eine der beweglichen Struktur (7, 8) und der festen Struktur (3-6) über eine Verbindung, die einen Drehfreiheitsgrad um eine erste Drehachse (A11) senkrecht zu der Längsrichtung (D1) definiert, ausgebildet ist,
- einen zweiten Teil (P2), der mit der Pleuelstange (57) über eine Verbindung verbunden ist, die einen Drehfreiheitsgrad um eine zweite Drehachse (A12) parallel zu der ersten Drehachse (A11) definiert, wobei die Pleuelstange (57) mit einer zweiten Struktur verbunden ist, die durch die andere der beweglichen Struktur (7, 8) und der festen Struktur (3-6) über eine Verbindung, die einen Drehfreiheitsgrad um eine dritte Drehachse (A13) parallel zu der zweiten Drehachse (A12) definiert, ausgebildet ist,
wobei die Pleuelstange (57) ein erstes Ende (81), das die zweite Drehachse (A12) definiert, und ein zweites Ende (82), das die dritte Drehachse (A13) definiert, umfasst, wobei das erste Ende (81) der Pleuelstange (57) auf einer Schwenkoberfläche (73) aufliegt, die durch den zweiten Teil (P2) des Hebelarms (55) ausgebildet ist, wobei das zweite Ende (82) der Pleuelstange (57) auf einer Schwenkoberfläche (75) aufliegt, die durch die zweite Struktur (5) ausgebildet ist.

2. Vorrichtung (1) nach Anspruch 1, wobei die durch den zweiten Teil (P2) des Hebelarms (55) ausgebildete Schwenkoberfläche (73) und die Schwenkoberfläche (75) der zweiten Struktur (5) relativ zueinander gegenüberliegend angeordnet sind.

3. Vorrichtung (1) nach Anspruch 1 oder 2, umfassend eine Achse (56), wie etwa einen Stift, die die erste Drehachse (A11) definiert, wobei diese Achse (56) mit der ersten Struktur (7, 8) fest verbunden ist.

4. Vorrichtung (1) nach Anspruch 3, wobei der erste Teil (P1) des Hebelarms (55) eine Schwenkoberfläche (71), die konfiguriert ist, um auf der Achse (56) zur Auflage zu kommen, umfasst.

5. Vorrichtung (1) nach Anspruch 4, wobei die durch den ersten Teil (P1) des Hebelarms (55) ausgebildete Schwenkoberfläche (71) zum Ausüben, auf die Achse (56), einer Kraft konfiguriert ist, die eine in eine erste Richtung (S1) entlang der Längsrichtung (D1) ausgerichtete Komponente aufweist, wobei die durch den zweiten Teil (P2) des Hebelarms (55) ausgebildete Schwenkoberfläche (73) zum Ausüben, auf die Verbindungsstange (57), einer Kraft konfiguriert ist, die eine in eine zweite Richtung (S2) entlang der Längsrichtung (D1) ausgerichtete Komponente aufweist.

6. Vorrichtung (1) nach einem der Ansprüche 1 bis 5, wobei das Betätigungssystem ein Antriebsteil (52) umfasst, der Hebelarm umfassend (55) einen dritten Teil (P3), der mit diesem Antriebsteil (52) über eine Verbindung verbunden ist, die einen Drehfreiheitsgrad um eine vierte Drehachse (A14) parallel zu der ersten Drehachse (A11) definiert.

7. Vorrichtung (1) nach Anspruch 6, wobei die erste Drehachse (A11), die zweite Drehachse (A12) und die vierte Drehachse (A14) in derselben Ebene liegen.

8. Vorrichtung (1) nach Anspruch 6 oder 7, wobei das Antriebsteil (52) entlang einer Bewegungsachse (A2) parallel zu der Längsrichtung (D1) translatorisch beweglich ist.

9. Vorrichtung (1) nach einem der Ansprüche 1 bis 8, wobei das Betätigungssystem einen Motor umfasst, der zum Versetzen des Hebelarms (55) in eine Drehung, gleichzeitig um die erste Drehachse (A11) und die zweite Drehachse (A12), konfiguriert ist.

10. Vorrichtung (1) nach Anspruch 9, die die Merkmale des Anspruchs 6 einschließt, wobei der Motor zum Versetzen des Hebelarms (55) in eine Drehung um die erste Drehachse (A11) und die zweite Drehachse (A12) mittels des Antriebsteils (52) konfiguriert ist.

11. Vorrichtung (1) nach einem der Ansprüche 1 bis 10, wobei die bewegliche Struktur (7, 8) die erste Struktur ausbildet und wobei die feste Struktur (3-6) die zweite Struktur ausbildet.

12. Vorrichtung (1) nach einem der Ansprüche 1 bis 11, die für die thermodynamische Charakterisierung eines Fluids vorgesehen ist, die Vorrichtung (1) umfassend eine Kammer (41), die geeignet ist, das Fluid aufzunehmen, wobei die bewegliche Struktur (7, 8) konfiguriert ist, um das Volumen der Kammer (41) verändern zu können.

## Claims

1. A device (1) comprising a fixed structure (3-6), a structure (7, 8) that is movable along a longitudinal direction (D1), and a system for actuating the movable structure (7, 8), **characterized in that** the actuating system comprises a lever arm (55) and a connecting rod (57), the lever arm (55) comprising:
- a first part (P1) connected to a first structure formed by one of the movable structure (7, 8) and the fixed structure (3-6) via a connection defining a degree of freedom in rotation about a first axis of rotation (A11) perpendicular to the longitudinal direction (D1),
- a second part (P2) connected to the connecting rod (57) via a connection defining a degree of freedom in rotation about a second axis of rotation (A12) parallel to the first axis of rotation (A11), the connecting rod (57) being connected to a second structure formed by the other of the movable structure (7, 8) and the fixed structure (3-6) via a connection defining a degree of freedom in rotation about a third axis of rotation (A13) parallel to the second axis of rotation (A12),
the connecting rod (57) comprising a first end (81) defining the second axis of rotation (A12) and a second end (82) defining the third axis of rotation (A13), the first end (81) of the connecting rod (57) resting on a pivot surface (73) formed by the second part (P2) of the lever arm (55), the second end (82) of the connecting rod (57) resting on a pivot surface (75) formed by said second structure (5).

2. The device (1) according to claim 1, wherein the pivot surface (73) formed by the second part (P2) of the lever arm (55) and the pivot surface (75) of the second structure (5) are arranged opposite one another.

3. The device (1) according to claim 1 or 2, comprising an axis (56)-such as a pin-defining the first axis of rotation (A11), this axis (56) being rigidly connected to said first structure (7, 8).

4. The device (1) according to claim 3, wherein the first part (P1) of the lever arm (55) comprises a pivot surface (71) configured to rest against said axis (56).

5. The device (1) according to claim 4, wherein the pivot surface (71) formed by the first part (P1) of the lever arm (55) is configured to exert on said axis (56) a force having a component oriented in a first direction (S1) along the longitudinal direction (D1), the pivot surface (73) formed by the second part (P2) of the lever arm (55) being configured to exert on the connecting rod (57) a force having a component oriented in a second direction (S2) along the longitudinal direction (D1).

6. The device (1) according to any of claims 1 to 5, wherein the actuating system comprises a drive member (52), the lever arm (55) comprising a third part (P3) connected to this drive member (52) via a connection defining a degree of freedom in rotation about a fourth axis of rotation (A14) parallel to the first axis of rotation (A11).

7. The device (1) according to claim 6, wherein the first axis of rotation (A11), the second axis of rotation (A12), and the fourth axis of rotation (A14) lie in the same plane.

8. The device (1) according to claim 6 or 7, wherein the drive member (52) is movable in a translational manner along an axis of displacement (A2) parallel to the longitudinal direction (D1).

9. The device (1) according to any of claims 1 to 8, wherein the actuating system comprises a motor configured to rotate the lever arm (55) simultaneously about the first axis of rotation (A11) and the second axis of rotation (A12).

10. The device (1) according to claim 9, including the features of claim 6, wherein the motor is configured to rotate the lever arm (55) about the first axis of rotation (A11) and the second axis of rotation (A12) via the drive member (52).

11. The device (1) according to any of claims 1 to 10, wherein the movable structure (7, 8) forms said first structure and wherein the fixed structure (3-6) forms said second structure.

12. The device (1) according to any of claims 1 to 11, intended for the thermodynamic characterization of a fluid, the device (1) comprising a chamber (41) capable of receiving the fluid, the movable structure (7, 8) being configured to be able to change the volume of the chamber (41).
